# EUROPEAN PATENT SPECIFICATION

(11) **EP 0 806 196 B1**
(45) Date of publication and mention of the grant of the patent: **07.08.2002**
(21) Application number: 97303060.4
(22) Date of filing: 06.05.1997
(51) Int. Cl.: A61F 13/15

(54) **Disposable absorbent garment of pants type**
Absorbierende, hosenähnliche Wegwerfwindel
Couche-culotte absorbante jetable

(30) Priority: 07.05.1996 JP 11259096
(43) Date of publication of application: 12.11.1997
(73) Proprietor: UNI-CHARM CORPORATION, Kawanoe-shi Ehime-ken (JP)
(72) Inventor: Soga, Hiroyuki, Kagawa-ken (JP); Otsuba, Toshifumi, Kawanoe-shi, Ehime-ken (JP)
(74) Representative: Kensett, John Hinton

(56) References cited:
- EP-A- 0 329 160
- EP-A- 0 450 541
- GB-A- 2 253 131

## Description

The present invention relates generally to a pull-on disposable absorbent garment such as a pants type disposable diaper, training pants, incontinence pants or the like.

Pull-on disposable absorbent garment are well known in which a waist-opening and a pair of leg-openings are provided with elastic members so as to provide edges of these openings with a stretchability. It is also known that elastic members are provided in a large extent defined between a waist-opening edge and a crotch region of an absorbent garment so that a location of the garment corresponding to the liquid-absorbent core may be tightly maintained in contact with the wearer's skin under a pressure exerted by the elastic members from an outer surface of the absorbent core. Such garment is disclosed, for example, in EP-A-487921 or in EP-A-0 329 160 and by Japanese Patent Application Laid-Open Publication No. Hei4-289201.

However, when the location of the garment corresponding to the absorbent core is elastically maintained in close contact with the wearer's skin in order to prevent leakage of body fluids, it gives an oppressive sense to the wearer's waist or stomach due to the elastic members and significantly degrades a feeling to wear the garment.

The present invention is based on the finding that the leakage of body fluids can be prevented by improving a fitness of the garment not over the entire extent of the absorbent core but only along at least a portion of a peripheral edge of the absorbent core and thereby an apprehension that a feeling to wear the garment might be degraded.

In view of the problem as has been described above, it is a principal object of the invention to improve a fitness of the garment to the wearer's body at least in the proximity of longitudinally opposite ends of the absorbent core.

The object set forth above is achieved, according to the invention, by on a pull-on disposable absorbent garment comprising a laminate of a liquid-absorbent core having longitudinally opposite ends and transversely opposite side edges, a liquid-permeable topsheet and a liquid-impermeable backsheet, said two sheets respectively covering opposite surfaces of said absorbent core and extending beyond said longitudinally opposite ends as well as beyond said transversely opposite side edges of said absorbent core, and said laminate having a front waist region, a rear waist region and a crotch region extending between said front and rear waist regions, wherein, with said laminate longitudinally folded in two sections, said front and rear waist regions are bonded to each other along transversely opposite side edges of the respective regions to define a waist-opening and a pair of leg-openings, and said front and rear waist regions inclusive of said waist-opening as well as said pair of leg-openings are provided with a plurality of elastic members extending circumferentially of these openings and longitudinally spaced apart one from another, said pull-on disposable absorbent garment being characterized by that:
at least said front waist region of said front and rear waist regions has a first waist subregion defined by each 20mm in maximum above and below a front end of said absorbent core and a second waist subregion defined between said first waist subregion and an upper edge of said leg-opening, wherein the respective elastic members arranged in said first and second waist subregions are substantially uniform as far as material as well as cross-sectional area are concerned, and a distance by which each pair of adjacent elastic members arranged in said first waist subregion are spaced apart from each other is dimensioned to be smaller than a distance by which each pair of adjacent elastic members arranged in said second waist subregion are spaced apart from each other.

According to another aspect of the invention, the respective elastic members provided in said first and second waist subregions are substantially uniform as far as an elongation stress is concerned.

According to still another aspect of the invention, the respective elastic members provided in said first waist subregion have elongation stresses lower than the elongation stresses of the respective elastic members provided in said second waist subregion.

According to further aspect of the invention, within a region of said absorbent core extending rearward from said front end by at least 10mm as measured in a longitudinal direction thereof, said absorbent core has a thickness gradually decreasing toward said front end.

The pull-on disposable absorbent garment provided by the invention allows the garment to be reliably maintained in tight contact with the wearer's skin in the proximity of the longitudinally opposite ends of the absorbent core and thereby to prevent discharged body fluids from leaking beyond these ends of the absorbent core without degrading a feeling to wear the garment, since the elastic members are more densely arranged in the proximity of the longitudinally opposite ends of the absorbent core than in the remaining regions. Furthermore, the garment according to the invention provides a good appearance of the garment worn since the thickness of the absorbent core in the proximity of the longitudinally opposite ends thereof is dimensioned to be gradually decreased toward said ends and thereby a difference in level between the absorbent core and the wearer's skin is minimized.
Fig. 1 is a perspective view showing a pull-on disposable diaper according to the invention as partially broken away; and
Fig. 2 is a sectional view taken along line II-II in Fig. 1.

Details of a pull-on disposable absorbent garment according to the invention will be better understood from the following description of a pull-on disposable diaper as a typical embodiment of the invention given hereunder in reference with the accompanying drawings.

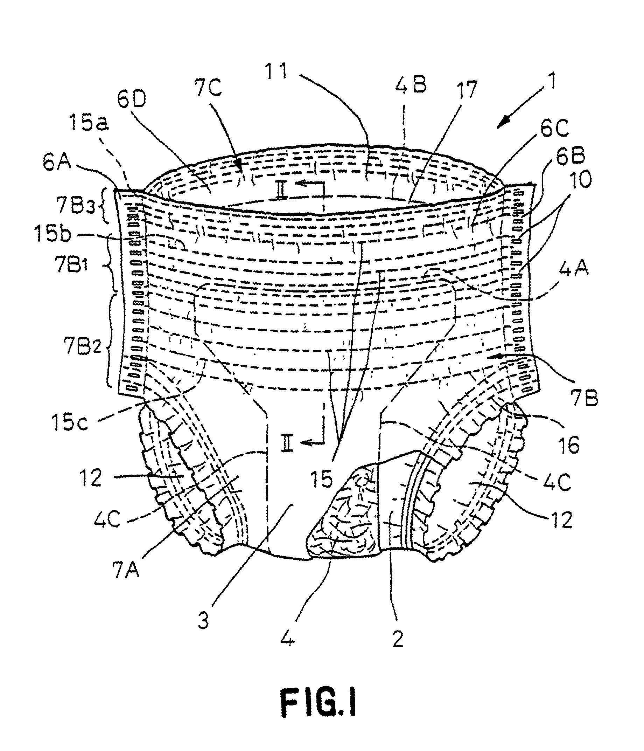

Referring to Fig. 1, a pull-on disposable diaper comprises a laminate 1. The laminate 1 comprises a liquid-permeable topsheet 2, a liquid-impermeable backsheet 3 and a liquid-absorbent core 4 disposed between these sheets 2, 3. The absorbent core 4 is hourglass- or I-shaped and has a peripheral edge longitudinally defined by front and rear ends 4A, 4B and transversely defined by opposite side edges 4C, 4C. The topsheet 2 and backsheet 3 are dimensioned to extending outward beyond the peripheral edge of the absorbent core 4 and bonded to each other along portions thereof extending outward beyond the peripheral edge of the absorbent core 4 so as to form transversely opposite side flaps 6A, 6B and longitudinally opposite end flaps 6C, 6D. Such laminate 1 can be divided into front and rear waist regions 7B, 7C with a relatively narrow crotch region 7A extending substantially in a middle between the front and rear waist regions 7B, 7C. The laminate 1 is longitudinally folded at the crotch region 7A in two sections with the topsheet 2 inside and then the front and rear waist regions 7B, 7C are bonded to each other along transversely opposite side edges thereof by bonding means 10 provided along these side edges so as to define a waist-opening 11 and a pair of leg-openings 12. The front waist region 7B inclusive of the waist-opening 11 and the pair of leg-openings 12 are respectively provided with circumferentially stretchable elastic members 15a, 15b, 15c; and 16.

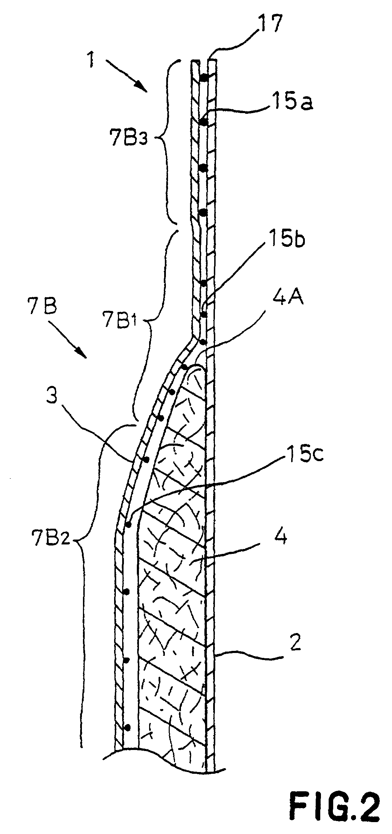

Referring now to Fig. 2, a portion of the absorbent core 4 occupying the front waist region 7B is so configured that, within a region of the absorbent core 4 including a front end 4A and extending rearward from the front end 4A by at least 10mm as measured in its longitudinal direction, the absorbent core 4 may have a thickness gradually decreasing toward the front end 4A. Such configuration may be obtained by gradually decreasing a quantity of the absorbent core forming material in this region toward the front end 4A or appropriately compressing the absorbent core forming material in this region. The portions of the topsheet 2 and backsheet 3 extending outward from the front end 4A are bonded to each other so as to form a waist-opening edge 17. Though not shown, it should be understood that the rear waist region 7C is also constructed in the same manner as the front waist region 7B.

The front waist region 7B is composed of a first waist subregion 7B₁ defined by each 20mm in maximum above and below the front end 4A of the absorbent core 4, a second waist subregion 7B₂ defined between the first waist subregion 7B₁ and an upper edge of the leg-opening 12, and a third waist subregion 7B₃ defined between the first waist subregion 7B₁ and the waist-opening edge 17. These waist subregions 7B₁, 7B₂, 7B₃ are respectively provided with a plurality of elastic members 15a, 15b, 15c extending in circumferential directions thereof and spaced apart one from another in a vertical direction thereof. These elastic members 15a, 15b, 15c are secured to an inner surface of the topsheet 2 or respective inner surfaces of the topsheet 2 and backsheet 3 in elastically extended conditions thereof. Each of the elastic members 15a provided in the third waist subregion 7B₃ has a cross-sectional area and an elongation stress larger and higher than each of the elastic members 15b, 15c provided in the first and second waist subregions 7B₁, 7B₂. While each of the elastic members 15b, 15c provided in the first and second waist subregions 7B₁, 7B₂ is substantially uniform so far as a cross-sectional area as well as an elongation stress are concerned, a distance by which each pair of adjacent elastic members 15b are spaced apart from each other in the first waist subregion 7B₁ is dimensioned to be smaller than a distance by which each pair of adjacent elastic members 15c are spaced apart from each other in the second waist subregion 7B₂ so that a surface pressure per unit area is larger in the first waist subregion 7B₁ than in the second waist subregion 7B₂. The elastic members 15b, 15c, 15a are rovided in the respective waist subregions 7B₁, 7B₂, 7B₃ with a 50% elongation stress of 5 ∼ 50g/150mm and an elongation ratio of 1.1 ∼ 2.5.

With the arrangement in which a surface pressure per unit area is higher in the first waist subregion 7B₁ than in the second waist subregion 7B₂ as has been described just above, the portion of the first waist subregion 7B₁ extending above and below the front end 4A of the absorbent core 4 is maintained in contact with the wearer's skin in spite of a rigidity of the absorbent core 4 during use of the diaper. In other words, this first waist subregion 7B₁ is advantageously maintained in contact with the wearer's skin more tightly than the second waist subregion 7B₂. However, such locally higher surface pressure will give the wearer a feeling of incompatibility. To eliminate such apprehension, it will be preferable that the first and socond waist subregions 7B₁, 7B₂ are substantially the same insofar as the surface pressure per unit area but the former provides a better fitness than the latter. This is achieved by an arrangement in which a distance by which each pair of adjacent elastic members 15b are spaced apart from each other in the first waist subregion 7B₁ is dimensioned to be smaller than a distance by which each pair of adjacent elastic members 15c are spaced apart from each other in the second waist subregion 7B₂ while each of the elastic members 15b in the second waist subregion 7B₁ has an elongation stress appropriately lower than each of the elastic members 15c in the second waist subregion 7B₂. Such adjustment of the elongation stress is achieved by an arrangement that each of the elastic members 15b in the first waist subregion 7B₁ has an elongation ratio smaller than each of the elastic members 15c in the second waist subregion 7B₂ while the elastic members 15b, 15c in the first and second waist subregions 7B₁, 7B₂, respectively, are substantially uniform as far as a material as well as a cross-sectional area are concerned or an arrangement that each of the elastic members 15b in the first waist subregion 7B₁ has a cross-sectional area appropriately smaller than each of the elastic members 15c in the second waist subregion 7B₂ while the elastic members 15b, 15c in the first and second waist subregions 7B₁, 7B₂ are substantially uniform as far as a material as well as an elongation ratio are concerned.

While a cross-section of the elastic member 15 may be either circular or rectangular, this cross-section is preferably dimensioned to have a diameter as measured in a direction of its width larger than a diameter as measured in a direction of its thickness.

While the description has been given above with respect to the front waist region 7B, it should be understood that the rear waist region 7C may have the arrangement similar to or more or less different from the arrangement of the front waist region 7B.

The diaper of the arrangement as has been described hereinabove reliably prevents discharged body fluids from leaking beyond the front end 4A of the absorbent core 4, since the elastic members 15 are densely arranged in the region just above and below the front end 4A of the absorbent core 4 which is reliably maintained in contact with the wearer's skin in the proximity of the front end 4A. The elastic members 15a provided in the third waist subregion 7B₃ extend along the waist line of the front and rear waist regions 7B, 7C substantially without a break.

It is possible to improve a fitness in the proximity of the front end 4A of the absorbent core by replacing the elastic members 15b by a single or plural relatively wide band-like elastic member(s) in the first waist subregion 7B₁. In this case, the band-like elastic member should have a moisture permeability sufficient to assure a breathability essential to the diaper.

For implementation of the invention, a nonwoven fabric or a perforated plastic film may be employed as the topsheet 2 and a plastic film such as a polyethylene film may be employed as the backsheet 3. As the absorbent core 4, fluff pulp or a mixture of fluff pulp and a hydrocolloid material such as superabsorbent polymer particles formed in a desired shape by the well known technique may be employed. A suitable material having a rubber elasticity such as rubber or plastic elastomer may be employed as the elastic members 15, 16.

Japanese Patent Application No. Hei8-112590 filed on May 7, 1996 (Laid-Open Publication No. Hei-9-84826) is hereby mentioned.

## Claims

1. A pull-on disposable absorbent garment comprising a laminate of a liquid-absorbent core (4) having longitudinally opposite ends (4A, 4B) and transversely opposite side edges (4C), a liquid-permeable topsheet (2) and a liquid-impermeable backsheet (3), said two sheets (2, 3) respectively covering opposite surfaces of said absorbent core and extending beyond said longitudinally opposite ends (4A, 4B) as well as beyond said transversely opposite side edges (4C) of said absorbent core, and said laminate having a front waist region (7B), a rear waist region (7C) and a crotch region (7A) extending between said front and rear waist regions, wherein, with said laminate longitudinally folded in two sections, said front and rear waist regions (7B, 7C) are bonded to each other along transversely opposite side edges of the respective regions to define a waist-opening (11) and a pair of leg-openings (12), and said front and rear waist regions inclusive of said waist-opening (11) as well as said pair of leg-openings (12) are provided with a plurality of elastic members (15a, 15b, 15c, 16) extending circumferentially of these openings and longitudinally spaced apart one from another, said pull-on disposable absorbent garment being **characterized in that**:
at least said front waist region (7C) of said front and rear waist regions has a first waist subregion (7B₁) defined by each 20mm in maximum above and below a front end (4A) of said absorbent core and a second waist subregion (7B₂) defined between said first waist subregion (7B₁) and an upper edge of said leg-opening (12), wherein the respective elastic members (15b, 15c) arranged in said first and second waist regions (7B₁, 7B₂) are substantially uniform as far as material as well as cross-sectional area are concerned, and a distance by which each pair of adjacent elastic members (15b) arranged in said first waist subregion (7B₁) are spaced apart from each other is dimensioned to be smaller than a distance by which each pair of adjacent elastic members (15c) arranged in said second waist subregion (7B₂) are spaced apart from each other.

2. The garment according to Claim 1, wherein the respective elastic members (15b, 15c) arranged in said first and second waist subregions (7B₁, 7B₂) are substantially uniform as far as elongation stress are concerned.

3. The garment according to Claim 1, wherein the respective elastic members (15b) arranged in said first waist subregion (7B₁) have an elongation stress lower than the elongation stress of the respective elastic members (15c) arranged in said second waist subregion (7B₂).

4. The garment according to Claim 1, 2 or 3, wherein, within a region of said absorbent core extending rearward from said front end (4A) by at least 10mm as measured in a longitudinal direction thereof, said absorbent core has a thickness gradually decreasing toward said front end (4A).

## Patentansprüche

1. Absorbierende Anzieh-Wegwerfwindel, die folgendes umfasst: ein Laminat eines flüssigkeitsabsorbierenden Kerns (4), der in Längsrichtung entgegengesetzte Enden (4A, 4B) und in Querrichtung entgegengesetzte seitliche Kanten (4C) aufweist, eine flüssigkeitsdurchlässige Deckschicht (2) und eine flüssigkeitsundurchlässige Trägerschicht (3), wobei genannte zwei Schichten (2, 3), die jeweils entgegengesetzte Flächen des genannten absorbierenden Kerns decken und sich über genannte in Längsrichtung entgegengesetzte Enden (4A, 4B) sowie über genannte in Querrichtung entgegengesetzte Kanten (4C) des genannten absorbierenden Kerns hinaus erstrecken, und genanntes Laminat eine vordere Taillienzone (7B), eine hintere Taillienzone (7C) und eine Schrittzone (7A) aufweist, die sich zwischen den genannten vorderen und hinteren Taillienzonen erstreckt, wobei, wenn das genannte Laminat in Längsrichtung in zwei Teile gefaltet ist, genannte vordere und hintere Taillienzonen (7b, 7C) miteinander, entlang der in Querrichtung entgegengesetzten seitlichen Kanten der jeweiligen Zonen, verbunden sind, um eine Taillienöffnung (11) und ein Paar Beinöffnungen (12) zu definieren, und genannte vordere und hintere Taillienzonen, einschließlich der genannten Taillienöffnung (11) sowie das genannte Paar von Beinöffnungen (12) mit einer Vielheit von elastischen Elementen (15a, 15b, 15c, 16) versehen sind, die sich am Umfang verlaufend und in Längsrichtung mit Abstand voneinander erstrecken, genannte absorbierende, Anzieh-Wegwerfwindel **dadurch gekennzeichnet ist, dass**:
wenigstens genannte vordere Taillienzone (7C) der genannten vorderen und hinteren Taillienzonen eine erste Taillienunterzone (7B₁) aufweist, die durch jeweils 20 mm Maximum oberhalb und unterhalb eines vorderen Endes (4A) des genannten absorbierenden Kerns definiert ist und eine zweite Taillienunterzone (7B₂) aufweist, die zwischen genannter ersten Taillienunterzone (7B₁) und einer oberen kante der genannten Beinöffnung (12) definiert ist, wobei die jeweiligen in genannten ersten und zweiten Taillienzonen (7B₁,7B₂) angeordneten elastischen Elemente (15b, 15c), im wesentlichen, soweit es das Material sowie den Querschnitt betrifft, gleich sind, und eine Distanz durch die jedes Paar benachbarter elastischen Elementen (15b) in genannter ersten Taillienunterzone (7B₁) mit Abstand voneinander angeordnet ist, so bemessen ist, dass sie kleiner als eine Distanz ist, durch die jedes Paar benachbarter elastischen Elemente (15c) in genannter zweiten Taillienunterzone (7B₂) mit Abstand voneinander angeordnet ist.

2. Kleidungsstück nach Anspruch 1, wobei die in genannten ersten und zweiten Taillienunterzonen (7B₁,7B₂) angeordneten jeweiligen elastischen Elemente (15b, 15c), soweit es Dehnungsbeanspruchung betrifft, im wesentlichen gleich sind.

3. Kleidungsstück nach Anspruch 1, wobei die jeweiligen, in genannter ersten Taillienunterzone (7B₁) angeordneten, elastischen Elemente (15b) eine Dehnungsbeanspruchung aufweisen, die niedriger als die Dehnungsbeanspruchung der jeweiligen elastischen Elemente (15c) ist, die in der genannten zweiten Taillienunterzone (7B₂) angeordnet sind.

4. Kleidungsstück nach Anspruch 1, 2 oder 3, wobei, innerhalb einer Zone des genannten absorbierenden Kerns, der sich ab genanntem vorderen Ende (4A) um mindestens 10 mm in Längsrichtung gemessen nach hinten erstreckt, genannter absorbierender Kern eine Dicke aufweist, die in Richtung des genannten vorderen Endes (4A) allmählich abnimmt.

## Revendications

1. Culotte absorbante jetable, comprenant un stratifié d'une partie centrale absorbant les liquides (4) ayant des extrémités opposées dans le sens de la longueur (4A, 4B) et des bords latéraux opposés dans le sens transversal (4C), un feuillet supérieur perméable aux liquides (2) et un feuillet arrière imperméable aux liquides (3), lesdits deux feuillets (2, 3), couvrant, respectivement, les surfaces opposées de ladite partie centrale absorbante et s'étendant au-delà desdites extrémités opposées dans le sens de la longueur (4A, 4B) ainsi que au-delà desdits bords opposés dans le sens transversal (4C) de ladite partie centrale absorbante, et ledit stratifié ayant une région de taille avant (7B), une région de taille arrière (7C), et une région d'entrejambe (7A) qui s'étend entre lesdites régions de taille avant et de taille arrière, dans laquelle, ledit stratifié étant replié en deux sections, dans le sens de la longueur, lesdites régions de taille avant et de taille arrière (7B, 7C) sont liées l'une à l'autre le long des bords latéraux opposés dans le sens transversal des régions respectives afin de définir une ouverture pour la taille (11) et une paire d'ouvertures pour les jambes (12), et lesdites régions de taille avant et de taille arrière, y compris ladite ouverture pour la taille (11), ainsi que ladite paire d'ouvertures pour les jambes (12), sont munies d'une pluralité d'éléments élastiques (15a, 15b, 15c, 16) qui s'étendent sur la circonférence de ces ouvertures et qui sont espacées l'une de l'autre, dans le sens de la longueur, ladite culotte absorbante jetable étant **caractérisée en ce que** :
au moins ladite région de taille avant (7C) desdites régions de taille avant et de taille arrière présente une première sous-région de taille (7B₁) définie par 20 mm au maximum au-dessus et en dessous d'une extrémité avant (4A) de ladite partie centrale absorbante, et une seconde sous-région de taille (7B₂) définie entre ladite première sous-région de taille (7B₁) et un bord supérieur de ladite ouverture pour la jambe (12), dans laquelle les éléments élastiques respectifs (15b, 15c) agencés dans lesdites première et seconde sous-régions de taille (7B₁, 7B₂) sont sensiblement uniformes aussi bien en ce qui concerne le matériau que la surface de section transversale, et **en ce que** la distance qui sépare chaque paire d'éléments élastiques adjacents (15b) agencés dans ladite première sous-région de taille (7B₁) est calculée pour être inférieure à la distance qui sépare chaque paire d'éléments élastiques adjacents (15c) agencés dans ladite seconde sous-région de taille (7B₂).

2. Culotte selon la revendication 1, dans laquelle les éléments élastiques respectifs (15b, 15c) agencés dans lesdites première et seconde sous-régions de taille (7B₁, 7B₂) sont sensiblement uniformes en ce qui concerne les contraintes d'allongement.

3. Culotte selon la revendication 1, dans laquelle les éléments élastiques respectifs (15b) agencés dans ladite première sous-région de taille (7B₁) ont une contrainte d'allongement inférieure à la contrainte d'allongement des éléments élastiques respectifs (15c) agencés dans ladite seconde sous-région de taille (7B₂).

4. Culotte selon la revendication 1, 2 ou 3, dans laquelle, à l'intérieur d'une région de ladite partie centrale absorbante qui s'étend vers l'arrière à partir de ladite extrémité avant (4A) d'au moins 10 mm, tel que mesuré dans le sens de la longueur de celle-ci, ladite partie centrale absorbante a une épaisseur qui diminue progressivement en direction de ladite extrémité avant (4A).
